# EUROPEAN PATENT SPECIFICATION

(11) **EP 3 711 716 B1**
(45) Date of publication and mention of the grant of the patent: **03.01.2024**
(21) Application number: 18877449.1
(22) Date of filing: 06.11.2018
(51) Int. Cl.: A61F 2/24

(54) **VALVE STENT, VALVE PROSTHESIS AND DELIVERY DEVICE**
KLAPPENSTENT, KLAPPENPROTHESE UND FREISETZUNGSVORRICHTUNG
ENDOPROTHÈSE DE VALVULE, PROTHÈSE DE VALVULE ET DISPOSITIF DE POSE

(30) Priority: 17.11.2017 CN 201711148589
(43) Date of publication of application: 23.09.2020
(73) Proprietor: Shanghai Microport Cardioflow Medtech Co., Ltd., Shanghai 201203 (CN)
(72) Inventor: SHI, Xingkun, Shanghai 201203 (CN); YANG, Ming, Shanghai 201203 (CN); CHEN, Guoming, Shanghai 201203 (CN); LI, Yu, Shanghai 201203 (CN)
(74) Representative: Lavoix
(86) International application number: PCT/CN2018/114215
(87) International publication number: WO 2019/096033

(56) References cited:
- EP-A1- 2 959 866
- WO-A1-2015/077599
- CN-A- 102 413 793
- CN-A- 103 190 968
- CN-A- 105 792 780
- US-A1- 2012 022 633
- US-B2- 8 317 858

## Description

### TECHNICAL FIELD

The present application relates to the technical field of medical instruments and, more specifically, to a valve stent, a valve prosthesis and a delivery device.

### BACKGROUND

With the aging of the global population, aortic valve disease has become one of the most common cardiovascular diseases, with an incidence of 2-5% in China and ranking as the third most frequent disease after coronary heart disease and hypertension in the United States and Europe. Every year, tens of thousands of patients benefit from surgical aortic valve replacement (SAVR). However, even in developed countries, there are still many patients with severe aortic valve disease who are inoperable due to a number of reasons such as late stages of the disease, advanced ages or multiple comorbidities. The debut of percutaneous artificial aortic valves, as well as the continuous performance improvement of such products, is undoubtedly a blessing for these patients because they provide an effective alternative for treating the disease.

Possible causes of aortic valve disease include birth defects, natural aging, infection, scarring, etc. Calcium may deposit around the aortic valve over time, which can narrow the aortic valve and/or make it close insufficiently, causing "aortic regurgitation". Most of patients with aortic valve disease suffer from angina, syncope and heart failure. These symptoms can lead to a serious decline in the quality of life and a significantly shortened survival time, effective treatment is thus necessary.

Since Cribier and colleagues first reported a first case performing transcatheter aortic valve replacement (TAVR) on human body in 2002, considerable effort has been devoted by researchers and physicians all over the world in basic and clinical studies in this art. These studies have achieved good clinical results and showed that this novel technique is safe and effective for patients who are inoperable or whose reception of surgical valve replacement is associated with a high risk. Compared with the surgical approach, percutaneous aortic valve replacement eliminates the need for open-heart procedures or for the support of an extracorporeal circulation machine and provides the advantages of minimal invasiveness, fewer complications, quick recovery, less patient suffering and high acceptance. Despite the fact that most TAVR-treated patients are high-risk ones, the treatment method can still achieve a 30-day survival rate of higher than 90% and significantly improve postoperative hemodynamic parameters.

After numerous modifications and improvements to percutaneous artificial aortic valves, representative heart valve prostheses currently used in clinical practice are Edwards and CoreValve systems.

An Edwards-SAPIEN prosthetic valve is formed of bovine pericardial tissue and is assembled by suturing onto a stent fabricated from stainless steel (or a cobalt-chromium alloy). The valve can be deployed at the valve annulus by a balloon-expandable stent in an anterograde, retrograde, or transapical manner without the need for use of a delivery sheath. The prosthetic valves for clinical use are available in sizes of 23 mm and 26 mm. Patent publication WO2009/149462A2 describes examples of such aortic valves. A large number of clinical trials have been conducted on Edwards-SAPIEN aortic valves and are very fruitful.

CoreValve systems are another kind of stented valves that have been successfully applied in clinical use (for the first time in human in 2005). CoreValve prosthetic valves are tri-leaflet porcine pericardial valves sutured onto self-expanding nitinol stents that are currently available in sizes of 26 mm, 29 mm and 31 mm. U.S. Pat. Pub US2011/0172765A1 provides examples of valves of this type. A stent of a CoreValve system is made of nickel-titanium memory alloy and typically has: a leading section with a relatively low radial strength, configured for anchoring to the ascending aorta above the sinus of Valsalva; a concave intermediate section affixed with leaflets, for avoiding obstruction of blood flow through the coronary arteries; and a trailing section with a relatively high radial strength, configured to be securely disposed in the aortic annulus. The latest clinical studies have proven good hemodynamic effects and a low 30-day mortality of 8%, which suggests their satisfactory safety profile.

Nevertheless, the existing valve prostheses employ upright attachment lugs, which fit against the ascending aortic wall in the deployed state in a human body. However, since they are bulkier than other mesh end portions, there is a risk of puncturing the ascending aorta due to their upright orientation. In addition, the loading of these upright attachment lugs is difficult and time-consuming and thus unfavorable to surgical procedures, in which time is highly valued and there is always the case that a period as short as even several seconds matters in saving a life. Therefore, from this perspective, such upright attachment lugs are significantly disadvantageous. Furthermore, the design of the upright attachment lugs is also not conducive to valve-in-valve (ViV) TAVR procedures because it may hinder the anchoring of the second valve.

WO2015/077599 A1 discloses an apparatus for replacing native heart valves with prosthetic heart valves, wherein a support frame configured to be implanted in a heart valve comprises an annular main body formed by a plurality of angled struts, the main body including a plurality of peaks formed by the intersection of respective adjacent struts. The support frame further comprises one or more leaflet-engaging mechanisms configured to engage leaflets of the heart valve. The support frame can be radially expandable and collapsible.

### SUMMARY OF THE INVENTION

It is an objective of the present application to overcome the above-discussed problems that is difficult and time-consuming in loading the conventional prostheses, by presenting a novel valve stent, valve prosthesis and delivery device.

To solve the above problem, the present application provides a valve stent according to claim 1.

Optionally, the angle at which the first bent section is bent and folded toward the interior of the stent body ranges from 10° to 45° and the angle at which the second bent section is bent and folded toward the interior of the stent body ranges from 5° to 40°.

Optionally, the angle at which the second bent section is bent and folded toward the interior of the stent body ranges from 15° to 30°.

Optionally, the first bent section comprises a connection rod and the second bent section comprises an attachment portion; the connection rod has one end connected to the stent body and a further end connected to the attachment portion; one end of the attachment portion which is farther away from the connection rod is an free end.

Optionally, the first bent section comprises a first connecting section and the second bent section comprises an attachment portion and a second connecting section connected to the attachment portion; the second connecting section is connected to one end of the first connecting section, a further end of the first connecting section is connected to the stent body, one of the attachment portion which is farther away from the second connecting section is a free end.

Optionally, the at least two attachment lugs have a same shape and are uniformly distributed circumferentially on the stent body.

Optionally, an attachment portion of each of the attachment lugs defines a through hole.

Optionally, each of the attachment lugs has a length ranging from 1 mm to 6 mm.

Optionally, the length of each of the attachment lugs ranges from 3 mm to 5 mm.

Optionally, the attachment lugs are fabricated through cutting or braiding.

The present application further provides a valve prosthesis, comprising an artificial valve and a valve stent as claimed in any one of claims 1 to 8, wherein the artificial valve is attached to the valve stent.

The present application further provides a delivery device, configured to deliver the valve prosthesis of claim 9, the delivery device comprising: a sheath configured to receive the valve prosthesis; an inner tube disposed inside the sheath; and an attachment member sleeved over and fixed to the inner tube, the attachment member configured to restrict positions of the attachment lugs in the valve prosthesis relative to the inner tube.

Optionally, further comprising a guide tube disposed outside the sheath, wherein the guide tube has a tapered inner surface and is able to be sleeved over the inner tube, the guide tube is configured to clamp the attachment lugs of the valve prosthesis against the attachment member.

Optionally, a plurality of recesses are formed in an outer circumferential surface of the attachment member in a uniform manner, and each of the recesses is configured to receive the attachment portion of a respective one of the attachment lugs.

Optionally, the recess comprises a bottom portion complementary in shape to the attachment portion.

Optionally, a protrusion is provided in the bottom portion of the recess, and the protrusion matches with a through hole defined in the attachment portion of the respective attachment lug in the valve prosthesis.

The valve stent, valve prosthesis and delivery device provided herein offer the following advantages:
First, bending and folding the attachment lugs toward the interior of the stent body can reduce the difficulty in inwardly deflecting the attachment lugs and securing them to the delivery device during the valve prosthesis's loading into the sheath of the delivery device and can thus shorten the time required for the loading of the valve stent, resulting in increased valve prosthesis loading efficiency.

Second, in each attachment lug, the angle at which the first bent section is bent and folded toward the interior of the stent body is greater than the angle at which the second bent section is bent and folded toward the interior of the stent body, and the first bent section is closer than the second bent section to the stent body, with respect to the axial direction of the stent body, thus additionally lowering the difficulty in inwardly deflecting the attachment lugs and securing them to the delivery device during the valve prosthesis's loading into the sheath of the delivery device, additionally shortening the time required for the loading of the valve stent and resulting in additional increase in valve prosthesis loading efficiency.

Third, bending and folding the attachment lugs toward the interior of the stent body can lower the risk of puncturing the ascending aortic wall by the attachment lugs.

Fourth, bending and folding the attachment lugs in valve prosthesis toward the interior of the stent body can limit the axial movement of the second valve prosthesis in a ViV procedure, reducing the risk of valve prosthesis displacement.

### BRIEF DESCRIPTION OF THE DRAWINGS

Fig. 1 is a front view of a valve prosthesis according to an embodiment of the present application.
Fig. 2 is a top view of a valve prosthesis according to an embodiment of the application.
Fig. 3 is a front view of a valve stent according to an embodiment of the invention.
Fig. 4 schematically illustrates an enlarged view of part of a valve prosthesis according to an embodiment of the application.
Fig. 5 is a schematic diagram showing the structure of a delivery device according to an embodiment of the application.
Fig. 6 is a schematic diagram of attachment lugs in a valve prosthesis attached to an attachment member in accordance with an embodiment of the application.
Fig. 7 schematically illustrates an enlarged view of part of an attachment member according to an embodiment of the application.
Fig. 8 is a schematic diagram showing the loading of a conventional valve prosthesis.
Fig. 9 is a schematic diagram showing the loading of a valve prosthesis according to an embodiment of the application.
Fig. 10 is a schematic diagram of a valve prosthesis implanted in the aorta in accordance with an embodiment of the application.
Fig. 11 schematically illustrates an enlarged view of portion A of the valve prosthesis of Fig. 10 that is implanted in the aorta.
Fig. 12 is a schematic diagram showing a structure formed during implantation of two valve prostheses in the aorta in accordance with an embodiment of the application.
Fig. 13 is a schematic diagram showing a further structure formed during implantation of two valve prostheses in the aorta in accordance with an embodiment of the application.
Fig. 14 is a schematic diagram showing a first possible configuration of portion B of the two valve prostheses implanted in the aorta shown in Fig. 13.
Fig. 15 is a schematic diagram showing a second possible configuration of portion B of the two valve prostheses implanted in the aorta shown in Fig. 13.
Fig. 16 is a front view of a valve prosthesis according to another embodiment of the application.
Fig. 17 schematically illustrates an enlarged view of part of an attachment lug according to a further embodiment of the application.
Fig. 18 is a schematic diagram showing two valve prostheses implanted in the aorta in accordance with a further embodiment of the application.
Fig. 19 is a front view of a valve prosthesis according to a further embodiment of the application.
Fig. 20 is a front view of a valve prosthesis according to still a further embodiment of the application.
Fig. 21 schematically illustrates an enlarged view of part of an attachment lug in a valve prosthesis according to still a further embodiment of the application.
Fig. 22 schematically illustrates an enlarged view of part of a delivery device according to still a further embodiment of the application.

List of reference signs:
100, a valve prosthesis;
200, an artificial valve; 210, a leaflet; 220, a skirt;
300, a valve stent;
310, a stent body; 311, an inflow section; 312, an intermediate section; 313, an outflow section; 314, a suture ring;
320, an attachment lug; 320a, a first connecting end; 321, an attachment portion; 322, a connection rod; 323, a through hole; 324, a first connecting section; 325, a second connecting section;
400, a delivery device;
410, a sheath;
420, an inner tube; 421, a first segment; 422, a second segment;
430, an attachment member; 431, a recess; 432, a bottom portion; 433, a protrusion;
440, a guide tube; 441, an inner surface; 442, a first end; 443, a second end;
δ ₁, a first angle; δ₂, a second angle; δ₃, a third angle; J1, a first arrow; J2, a second arrow; 13, a third arrow; D, the aorta; W1, a first position; W2, a second position; P, a strut node; W3, a third position; W4, a fourth position;
500, a first valve prosthesis; and 600, a second valve prosthesis.

### DETAILED DESCRIPTION

Specific embodiments of the valve stent, valve prosthesis and delivery device proposed herein will be described in greater detail below with reference to the accompanying drawings. It is noted that the figures are provided in a very simplified form not necessarily presented to scale, with their only intention to facilitate convenience and clarity in explaining the disclosed embodiments. In the following description, for any attachment lug, attachment portion, connection rod, first connecting section or second connecting section, an angle at which it is bent and folded toward the interior of a stent body refers to an angle between it and a line parallel to a center axis of the valve stent.

### Embodiment 1

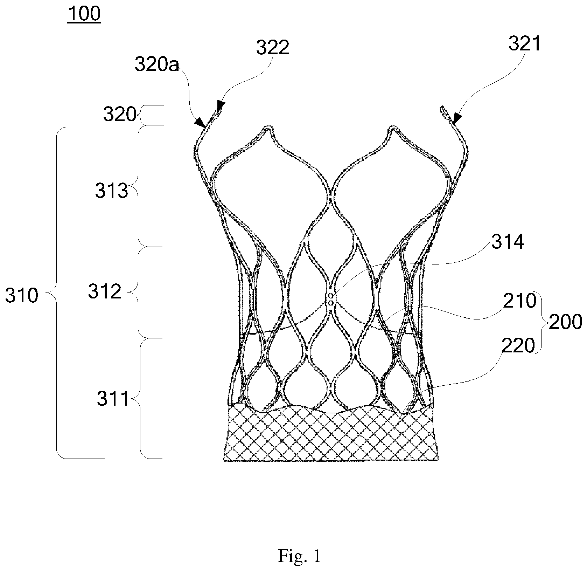

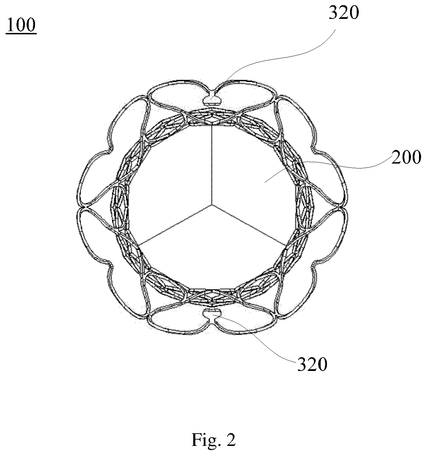

In this embodiment, there is provided a valve prosthesis. Reference is made to Fig. 1, a front view of the valve prosthesis, and to Fig. 2, a top view thereof. The valve prosthesis 100 includes an artificial valve 200 and a valve stent to which the artificial valve 200 is attached.

The artificial valve 200 includes leaflets 210 and a skirt 220, which are sewn on the valve stent. The skirt 220 is adapted to prevent perivalvular leakage. In this embodiment, the artificial valve 200 may be a tricuspid valve.

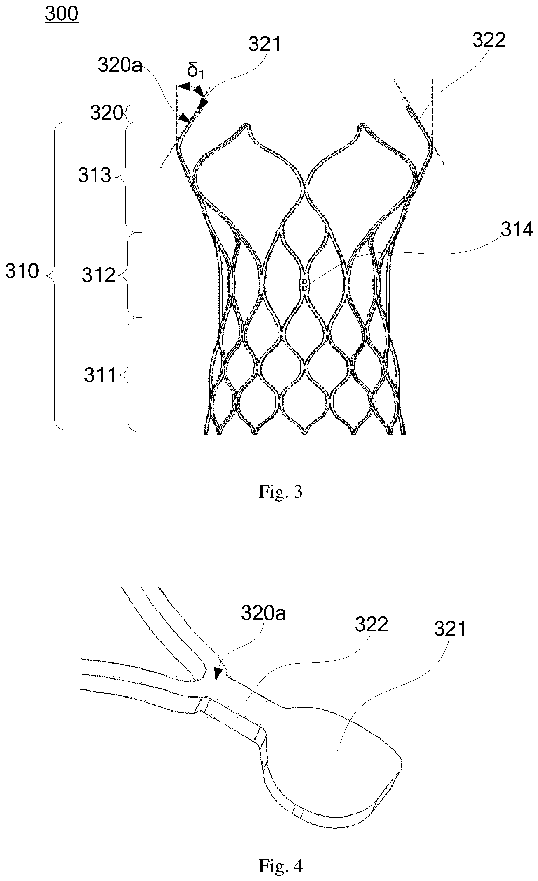

Reference is now made to Fig. 3, a front view of the valve stent. The valve stent 300 includes a stent body 310 and two attachment lugs 320. The stent body 310 takes the form of a tubular mesh, and the two attachment lugs 320 are provided on the stent body 310 in such a manner that they are both bent and folded toward the interior of the stent body 310. In particular, each attachment lug 320 has a first connecting end 320a connected to the stent body 310. The attachment lug 320 extends towards a center axis of the stent body 310 from the first connecting end 320a. In other words, the first end 320a is the portion of the attachment lug 320 that is located farthest away from the center axis of the stent body 310.

Referring to Fig. 3, the stent body 310 includes an inflow section 311, an intermediate section 312 joined to the inflow section 311, and an outflow section 313 joined to the intermediate section 312.

Specifically, the maximum outer diameter of the outflow section 313 is the largest, while the maximum outer diameter of the intermediate section 312 is the smallest, with the inflow section 311 having an outer diameter gradually decreasing from the end distal from the intermediate section 312 to the opposing end proximal to the intermediate section 312. The outflow section 313 is sparsely meshed and has large mesh openings, while the inflow section 311 is densely meshed and has small mesh openings. The outflow section 313 has less mesh openings than both the intermediate and inflow sections 312, 311. Those skilled in the art would appreciate that the outer diameters of the inflow, intermediate and outflow sections 311, 312, 313 in this embodiment may vary so that, for example, a middle portion of the inflow section 311 defines the maximum outer diameter thereof. Those skilled in the art would also appreciate that the numbers of mesh openings in the inflow, intermediate and outflow sections 311, 312, 313 in this embodiment may also vary so that, for example, they are equal or not to one another.

The intermediate section 312 defines a plurality of suture rings 314, on which the leaflets 210 are sutured. Preferably, the suture rings 314 are uniformly distributed circumferentially around the valve stent 300. Preferably, there are three suture rings 314 in the intermediate section 312.

The skirt 220 is sewn on the inflow section 311. The skirt 220 may be formed of a material with a sealing effect and for medical use and sewn using any suitable conventional technique.

With reference to Fig. 3, an end portion of the outflow section 313 distal from the intermediate section 312 is bent and folded toward the interior of the outflow section 313. In particular, the outflow section 313 extends outwardly with respect to the center axis of the stent body 310 from its junction with the intermediate section 312 to the portion of the outflow section 313 having the maximum outer diameter and then extends toward the center axis of the stent body 310.

In this embodiment, each attachment lug 320 may be shaped by a mold so that it is generally inclined toward the interior of the stent body 310. The attachment lug 320 is bent and folded toward the interior of the stent body 310 at an angle corresponding to an angle between the attachment lug 320 and a line extending parallel to the center axis of the valve stent 300. For example, as shown in Fig. 3, the angle between the attachment lug 320 and a line extending parallel to the center axis of the valve stent 300 may be defined as a first angle δ1. The angle at which the attachment lug 320 is bent and folded toward the interior of the stent body 310 ranges from 10° to 45°. Experimental studies have shown that when the angle at which the attachment lug 320 is bent and folded toward the interior of the stent body 310 exceeds 45°, the loading of the valve prosthesis 100 will become very difficult. On the other hand, when the angle at which the attachment lug 320 is bent and folded toward the interior of the stent body 310 is less than 10°, the bending and folding of the attachment lug 320 toward the interior of the stent body 310 will be insufficient to allow the prosthesis to offer the benefits as discussed herein. Thus, in this embodiment, each of the multiple attachment lugs 320 is bent and folded toward the interior of the stent body 310 preferably at an angle in the range of 10-45°.

In particular, with reference to Figs. 1 to 3, the two attachment lugs 320 are arranged at the end of the outflow section 313 distal from the intermediate section 312 in symmetry with respect to the center axis of the valve stent 300. In other embodiments, three, four or five attachment lugs 320 may be provided. Preferably, there are two to four attachment lugs 320. These attachment lugs 320 are preferred to be uniformly distributed circumferentially around the stent body 310. For example, when two attachment lugs 320 are included, they are spaced apart from each other by an angle of 180°. In case of three attachment lugs 320 being used, any adjacent two of them are spaced apart from each other by an angle of 120°. Of course, in other embodiments, the attachment lugs 320 may also be distributed circumferentially around the stent body 310 in a non-uniform manner. Preferably, the attachment lugs 320 have the same shape.

Specifically, reference is now made to Fig. 4, an enlarged view of part of the valve prosthesis. In this embodiment, each attachment lug 320 includes an attachment portion 321 serving as a first bent section and a connection rod 322 serving as a second bent section. The connection rod 322 is connected to the stent body 310 at one end and connected to the attachment portion 321 at the other end. The aforementioned first connecting end 320a corresponds to the end where the connection rod 322 connects the stent body 310. The end of the attachment portion 321 distal from the connection rod 322 forms a free end of the attachment lug 320. The attachment portion 321 is preferably flat in shape. Specifically, with reference to Figs. 1 to 4, the first end 320a is preferably connected to an apex of a mesh opening in the outflow section 313, and the attachment portion 321 has rounded smooth corners. The connection rod 322 is a rod-shaped component overall narrower than the attachment portion 321.

Preferably, the first bent section is bent and folded toward the interior of the stent body 310 at an angle ranging from 10° to 45°, while the second bent section is bent and folded toward the interior of the stent body 310 at an angle ranging from 5° to 40°. More preferably, the second bent section is bent and folded toward the interior of the stent body 310 at an angle ranging from 15° to 30°.

Each attachment lug 320 preferably has a length in the range of 1 mm to 6 mm. In addition, each attachment lug 320 may have a length ranging from 3 mm - 5mm. A length in such a range can facilitate the attachment lug 320 to achieve the above-discussed bending angles and hence better loading performance.

In this embodiment, the attachment lugs 320 are integral with the stent body 310. For example, the stent body 310 may be fabricated by cutting a shape memory alloy (typically, nitinol) tube into a tubular mesh with the attachment lugs 320 and shaping it using a mold. Of course, in other embodiments, the attachment lugs 320 and the stent body 310 may be fabricated separately and then fixed to each other by welding, sewing, etc.

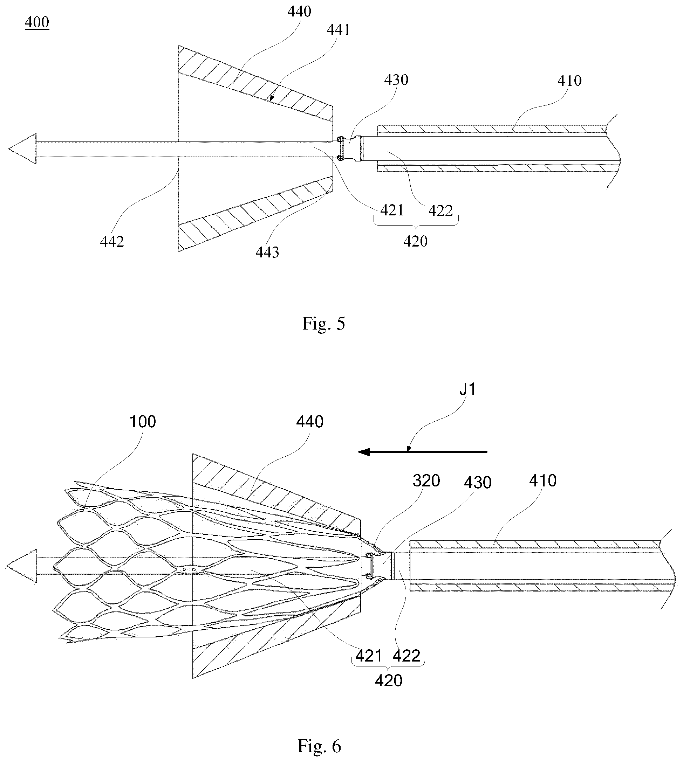

The valve prosthesis 100 according to this embodiment may be implanted in the body of a patient by using a delivery device. Prior to the implantation, the valve prosthesis 100 is usually loaded into the delivery device. Reference is now made to Fig. 5, a schematic diagram showing the structure of a delivery device according to an embodiment of the application. The delivery device 400 includes a sheath 410, an inner tube 420, an attachment member 430 and a guide tube 440. The sheath 410 is configured to receive the valve prosthesis 100, and the inner tube 420 is disposed within the sheath 410. The attachment member 430 is sleeved over the inner tube 420. The attachment member 430 serves to restrict the positions of the attachment lugs 320 in the valve prosthesis 100 relative to the inner tube 420. The attachment member 430 and the inner tube 420 can move along the sheath 410. The guide tube 440 is disposed outside the sheath 410 and adapted to clamp the attachment lug 320 of the valve prosthesis 100 against the attachment member 430.

Reference is again made to Fig. 5, and additionally to Fig. 6, a schematic diagram of attachment lugs in a valve prosthesis attached to an attachment member in accordance with an embodiment of the application. The inner tube 420 is composed of a first segment 421 and a second segment 422. In the scenario shown in Figs. 5 and 6, the attachment member 430 is arranged on the first segment 421.

The attachment member 430 is preferably disposed at the middle of the inner tube 420, i.e., at the junction between the first and second segments 421, 422. Referring to Figs. 5 and 6, in this embodiment, the first segment 421 is preferred to have an outer diameter smaller than that of the second segment 422. Preferably, the first segment 421 is fabricated from a material softer than that of the second segment 422.

At least two recesses 431 are formed in an outer circumferential surface of the attachment member 430. Preferably, these recesses 431 are uniformly distributed across the outer circumferential surface of the attachment member 430.

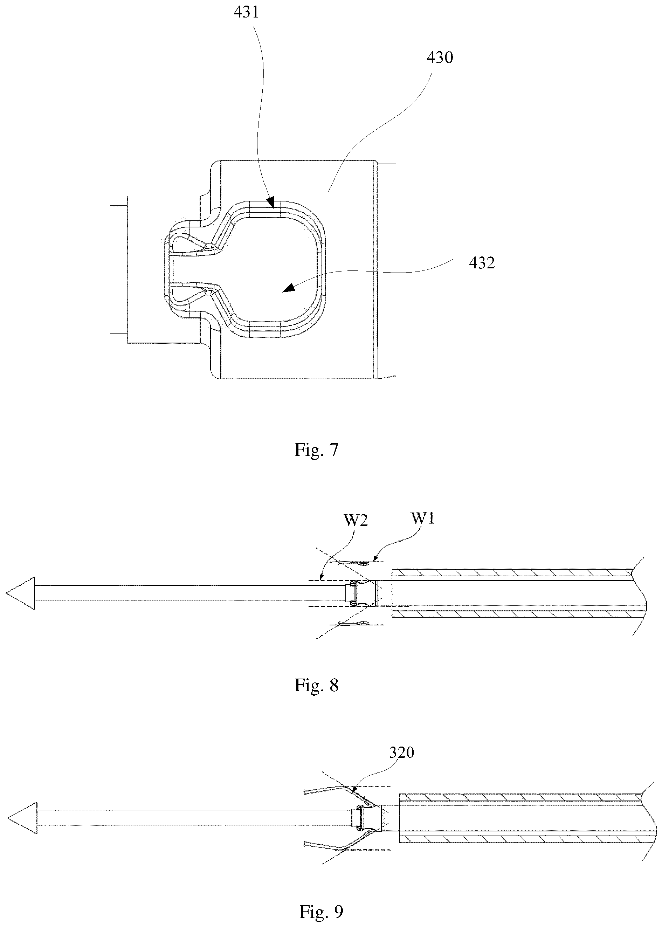

Reference is made to Fig. 7 which schematically illustrates an enlarged view of part of an attachment member according to an embodiment of the application. Each of the recesses 431 can receive the attachment portion 321 of a respective one of the attachment lugs 320. The recess 431 may have a bottom portion 432 complementary in shape to the attachment portion 321. For example, the bottom portion 432 of the recess 431 may define a first curved surface, with the inner side of the attachment portion 321 of the attachment lug 320 defining a second curved surface, which may partially coincide with the first curved surface, resulting in a fit between the two curved surfaces.

Preferably, the recess 431 has a depth that is greater than a thickness of the attachment lug 320 by 0-0.4 mm.

Referring to Figs. 6 and 7, the end of the attachment member 430 distal from the second segment 422 has an outer contour having a diameter longer an outer diameter of the first segment 421, defining a flange over the outer surface of the first segment 421. During loading of the valve prosthesis 100 onto the attachment member 430, the end of the outflow section 313 distal from the intermediate section 312 can abut against the flange so that the valve prosthesis 100 cannot further move relative to the inner tube 420. This is helpful in loading the valve prosthesis 100 into the sheath 410.

As shown in Figs. 5 and 6, the guide tube 440 has a tapered inner surface 441, as well as a first end 442 and a second end 443. The first end 442 is the end of the guide tube 440, where the inner surface 441 has a greater diameter, while the second end 443 is the end of the guide tube 440, where the inner surface 441 has a smaller diameter. In the configuration where the guide tube 440 is clamping the attachment lugs 320 in the valve prosthesis 100 against the attachment member 430, the guide tube 440 is sleeved over the inner tube 420, with the attachment lugs 320 in the valve prosthesis 100 being inserted through the end of the guide tube 440 where the inner surface 441 has a smaller diameter and thus compressed by the inner surface 441 of the guide tube 440.

A process to load the valve prosthesis 100 into the sheath 410 of the delivery device 400 will be described below.

At first, the valve prosthesis 100 is submerged in ice water, and the guide tube 440 is sleeved over the valve prosthesis 100 so that the attachment lugs 320 in the valve prosthesis 100 are compressed by the guide tube 440.

Next, the inner tube 420 is inserted into the valve prosthesis 100 and guide tube 440 from the side of the valve prosthesis 100 where the attachment lugs 320 are provided, so that the valve prosthesis 100 and guide tube 440 are sleeved over the inner tube 420.

After that, the positions of the valve prosthesis 100 and guide tube 440 are adjusted to align the attachment lugs 320 in the valve prosthesis 100 with the respective recesses 431 in the attachment member 430, and the guide tube 440 is advanced toward the sheath 410 so that the attachment lugs 320 are further compressed. Here, the direction in which the guide tube 440 is advanced is opposite to the horizontal direction indicated by the first arrow J1 in Fig. 6. As a result, the attachment lugs 320 are confined in the recesses 431 in the attachment member 430. At this time, the attachment lugs 320 in the valve prosthesis 100 come into contact with the bottom portions 432 of the recesses 431 in the attachment members 430. Alternatively, the end of the outflow section 313 distal from the intermediate section 312 may be first abutted against the flange defined by the attachment member 430, and the guide tube 440 and valve prosthesis 100 may be then turned to refine the positions of the attachment lugs 320.

Afterward, the sheath 410 is advanced toward the guide tube 440 so that the valve prosthesis 100 is gradually received in the sheath 410. The direction in which the sheath 410 is advanced is as indicated by the first arrow J1 in Fig. 6.

During the loading of the valve prosthesis 100 into the sheath 410, since the attachment lugs 320 are bent and folded toward the interior of the stent body 310, they can be easily deflected and secured to the delivery device 400, resulting in savings of time and thus improved efficiency in loading the valve stent 300.

Compared to the conventional valve prostheses employing upright attachment lugs 320, the valve prosthesis 100 in this embodiment can be much more easily loaded. A comparison will be made between the two-attachment lug design with reference to Fig. 8, a schematic diagram illustrating the loading of a conventional valve prosthesis including two upright attachment lugs 320, and to Fig. 9, a schematic diagram illustrating the loading of a valve prosthesis according to an embodiment of the present application. At the same amount of compression that causes the inventive attachment lugs to get close or even fit in the respective recesses in the attachment members, the two attachment lugs in the conventional valve prosthesis cannot be received in the sheath due to their expansions along the axis of the valve prosthesis, and the valve prosthesis must be additionally contracted in order to allow the attachment lugs to get closer to the recesses in the attachment member and then completely fit at their inner sides against the bottom portions of the recesses, i.e., shifting from the first positions W1 to the second positions W2. This requires the valve prosthesis to be crimped to a size smaller than or equal to an inner diameter of the sheath, which is often difficult to achieve in practice. Therefore, the valve prosthesis 100 according to this embodiment enables a significant decrease in loading complexity and a remarkable increase in loading efficiency.

In this embodiment, the valve prosthesis 100 can assume either a released or compressed configuration. In the expansion configuration, the valve prosthesis 100 expands to resemble a flower. In an implantation process, the valve prosthesis 100 can be compressed into the sheath 410, which has a very short inner diameter, of the delivery device 400 and delivered therein to the aortic root via an artery or the cardiac apex. The valve prosthesis 100 is then released and expands, unfolding the leaflets 210, so that it starts to function in place of the native valve.

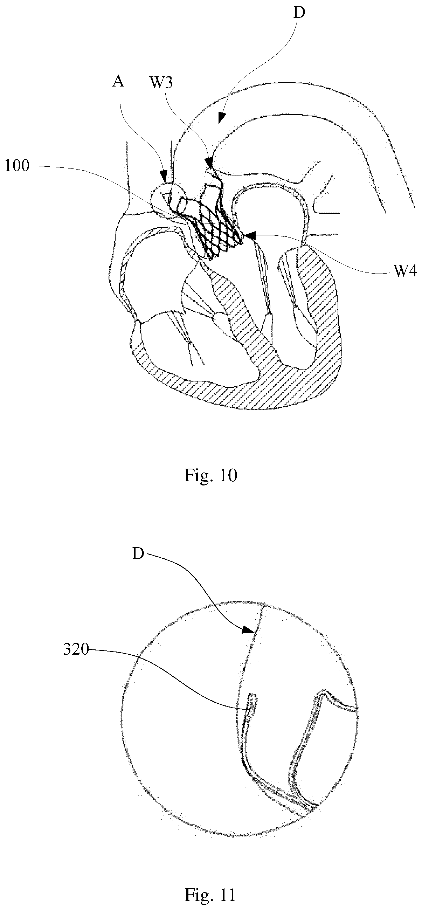

Specifically, reference is now made to Fig. 10, a schematic diagram of the valve prosthesis implanted in the aorta. During the implantation, after the valve prosthesis 100 is loaded in the sheath 410, it can be delivered by the delivery device 400 to a third position W3 in the root of the aorta D. After it has been confirmed that the valve prosthesis 100 is suitably positioned, the valve prosthesis 100 can be released. The release may proceed gradually, leading to gradual expansion of the valve prosthesis 100. After the completion of the release, the outflow section 313 of the valve prosthesis 100 will be located in a fourth position W4 in the ascending aorta D, as shown in Fig. 10.

In case of the valve prosthesis 100 being implanted in the root of the aorta D, the outflow section 313 of the valve prosthesis 100 will be located in the ascending aorta D. Since the aorta D is very elastic, the outflow section 313 that provides the greatest outer diameter of the valve prosthesis 100 will expand the aorta D. Because the attachment lugs 320 in the valve prosthesis 100 are bulkier and longer than the apices of the other mesh openings, if they are oriented upright, i.e., extending parallel to the center axis of the stent body, the attachment lugs are likely to puncture or damage the wall of the ascending aorta D and thus cause serious complications. Such a risk can be reduced by bending and folding the attachment lugs 320 toward the interior of the stent body 310. As can be seen from Fig. 11 which schematically illustrates an enlarged view of portion A of the valve prosthesis of Fig. 10 implanted in the aorta, clearances are formed between the attachment lugs 320, which is bent and folded toward the interior of the stent body 310, and the wall of the ascending aorta D, which can effectively avoid possible damage to the wall of the aorta D resulting from the attachment lugs 320.

After the implantation of the valve prosthesis 100 in the aorta D, it often happens that the positioning of the valve prosthesis 100 is unsatisfactory and tends to cause severe aortic regurgitation. In this case, the implantation of another valve prosthesis 100, i.e., a valve-in-valve (ViV) procedure, may be necessary. In such a ViV procedure, the positioning of the second valve prosthesis 100 mainly resort to support provided by outflow section 313 thereof.

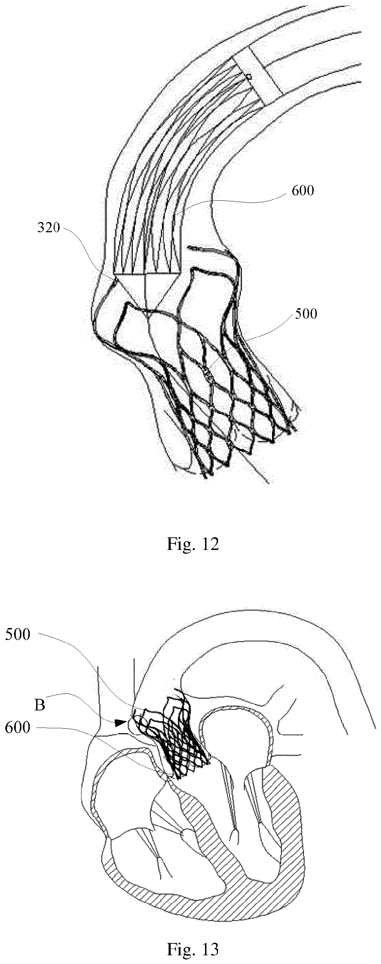

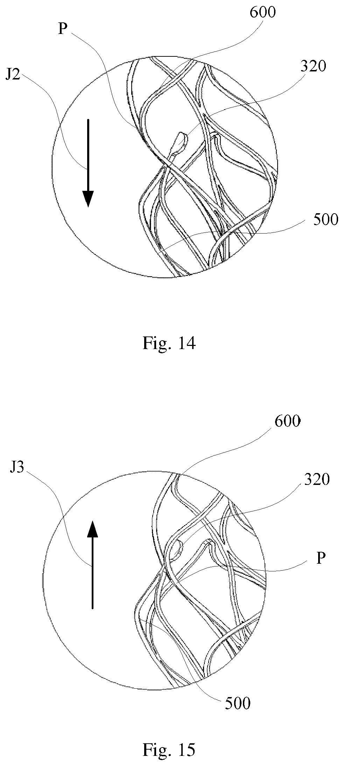

Reference is now made to Fig. 12, a schematic diagram showing a structure formed during implantation of two valve prostheses in the aorta in accordance with an embodiment of the application, which shows a valve prosthesis has already been implanted while a further valve prosthesis is in process of implantation, Fig. 13, a schematic diagram showing a further structure in which two valve prostheses are implanted in the aorta in accordance with an embodiment of the application, which shows both the two valve prosthesis have been implanted, Fig. 14, a schematic diagram showing a first possible configuration of portion B of the two valve prostheses implanted in the aorta shown in Fig. 13, and Fig. 15, a schematic diagram showing a second possible configuration of portion B of the two valve prostheses implanted in the aorta shown in Fig. 13, each of the valve prostheses 500 and 600 may be any implementation of the valve prosthesis 100 as discussed above, and a detailed description thereof will be omitted here.

The second valve prosthesis 600 is implanted subsequent to the implantation of the first valve prosthesis 500 in the aorta D, and is located in the first valve prosthesis 500. When the second valve prosthesis 600 is delivered to the root of the aorta D, as shown in Fig. 12, the second valve prosthesis 600 has not been released. The second valve prosthesis 600 may be adjusted to a suitable position relative to the aorta D and the first valve prosthesis 500 based on image information of the two valve prostheses displayed on a display device, before it is released. In addition, the sheath 410 may be turned to stagger the attachment lugs 320 of the second valve prosthesis 600 from those of the first valve prosthesis 500 by an angle of preferably 30°, followed by the release of the second valve prosthesis 600.

Since the attachment lugs 320 are bent and folded toward the interior of the stent body 310, when the second valve prosthesis 600 is completely released, there are at least two possible configurations of the two valve prostheses 100 (i.e., the first valve prosthesis 500 and the second valve prosthesis 600), as detailed below. In the first possible configuration shown in Fig. 14, the second valve prosthesis 600 is implanted from above the first valve prosthesis 500 so that each attachment lug 320 of the first valve prosthesis 500 is inserted into the second valve prosthesis 600 below a strut node P of the second valve prosthesis 600, i.e., on the side of the strut node P closer to the inflow section 311. Moreover, the attachment lugs 320 in the first valve prosthesis 500 are bent and folded toward the interior of the stent body 310, movement of the second valve prosthesis 600 away from the aorta D, as indicated by the second arrow J2 in Fig. 14, can thus be limited.

In the second possible configuration shown in Fig. 15, the second valve prosthesis 600 is implanted from below the first valve prosthesis 500 so that each attachment lug 320 in the first valve prosthesis 500 is inserted into the second valve prosthesis 600 above a strut node P of the second valve prosthesis 600, i.e., on the side of the strut node P farther away from the inflow section 311. Moreover, the attachment lugs 320 in the first valve prosthesis 500 are bent and folded toward the interior of the stent body 310, movement of the second valve prosthesis 600 toward the aorta D, as indicated by the third arrow J3 in Fig. 15, can thus be limited.

As noted above, the bending and folding of the attachment lugs 320 in the valve prosthesis 100 toward the interior of the stent body 310 can limit the degree of freedom for axial movement of the second valve prosthesis 600 implanted in a ViV procedure and thus greatly reduce the risk of displacement of the valve prosthesis 100.

### Embodiment 2

On the basis of the above embodiments, the applicant also proposes a valve prosthesis, which is overall similar to the above valve prosthesis, but differs therefrom in the shape and structure of the attachment lugs. The similarities will not be described again below, and the following description focuses on the structure of the attachment lugs.

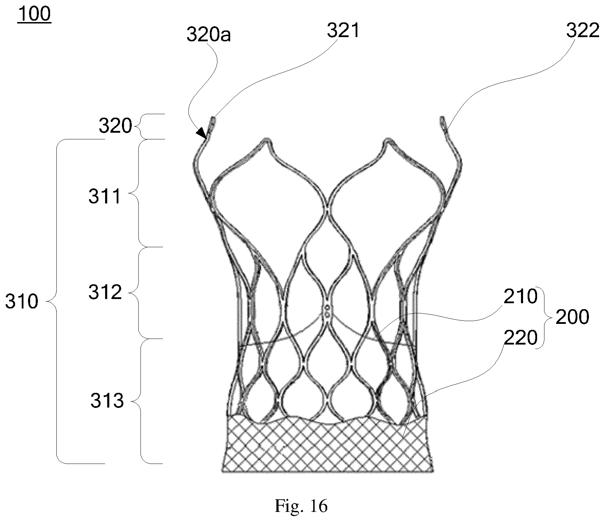

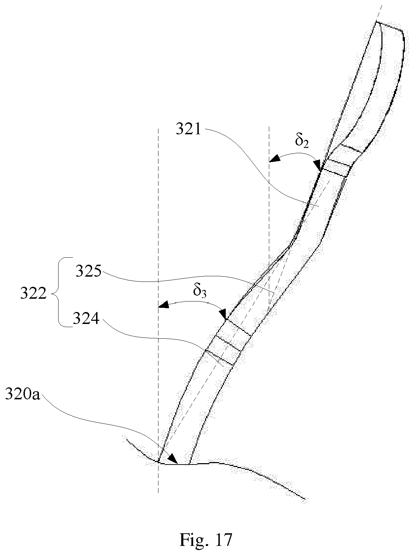

Reference is now made to Fig. 16, a front view of the valve prosthesis, and Fig. 17, an enlarged view of part of one attachment lug. In this embodiment, each attachment lug also includes a connection rod and an attachment portion, but differs in that different sections of each attachment lug may be bent and folded toward the interior of the stent body at different angles.

In this embodiment, the connection rod 322 may be a curved shaft, and the attachment portion 321 extends toward the center axis of the stent body 310 at an angle that is less than an angle between a line connecting both ends of the connection rod 322 and a line parallel to the center axis of the stent body 310. In other words, the angle at which the attachment portion 321 is bent and folded toward the interior of the stent body 310 is less than the angle at which the connection rod 322 is bent and folded toward the interior of the stent body 310. Here, as shown in Fig. 17, the angle at which the attachment portion 321 is bent and folded toward the interior of the stent body 310 is defined as a second angle δ2, and the angle at which the connection rod 322 is bent and folded toward the interior of the stent body 310 as a third angle δ3.

If all the portions of each attachment lug 320 are bent and folded toward the interior of the stent body 310 at the same angle, when the valve prosthesis is compressed during loading, for each attachment lug 320, only an end portion of the attachment portion 321 will fit against the respective recess 431 in the attachment member 430. Therefore, as will be appreciated by those skilled in the art, for each attachment lug 320, the attachment portion 321 only has a small contact area with the recess 431. As a result, during further operations for loading the valve prosthesis into the sheath 410, the attachment portions 321 of the attachment lugs 320 is likely to dislodge from the respective recesses 431. By contrast, if the attachment portion 321 of each attachment lug 320 extends toward the center axis of the stent body 310 at an angle, i.e., the angle at which the attachment portion 321 of each attachment lug 320 is bent and folded toward the interior of the stent body 310, less than an angle between a line connecting both ends of the connection rod 322 and a line parallel to the center axis of the stent body 310, the attachment portion 321 of each attachment lug 320 can have a larger contact area with the respective recess 431. In this way, dislodgement of the attachment portions 321 of the attachment lugs 320 from the respective recesses 431 during the loading of the valve prosthesis into the sheath can be avoided, resulting in an additional decrease in loading complexity and an additional increase in loading efficiency.

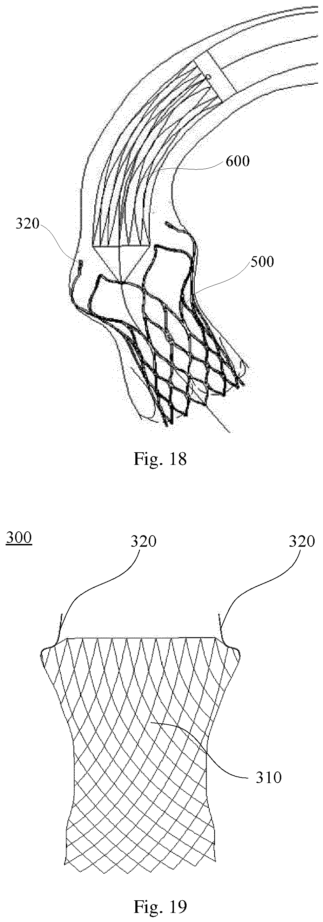

Additionally, if the connection rod 322 and attachment portion 321 of each attachment lug 320 are both bent and folded toward the interior of the stent body 310, the minimum inner diameter defined by the attachment lugs 320 will experience a decrease when the first valve prosthesis 500 is released. As a result, during the implantation of the second valve prosthesis 600, the attachment lugs 320 is likely to interact with the second valve prosthesis 600 or with the delivery device configured to deliver the second valve prosthesis 600, which may cause displacement of the first valve prosthesis 500, and thus complications such as conduction block, regurgitation, etc. In order to avoid this, as shown in Fig. 18, a schematic diagram showing two valve prostheses implanted in the aorta according to this embodiment, in each of these valve prostheses, the attachment portion 321 of each attachment lug 320 is bent and folded toward the interior of the stent body 310 at an angle less than an angle between a line connecting both ends of the connection rod 322 and a line parallel to the center axis of the stent body 310. With this arrangement, excessive approaching of the two attachment lugs 320 on the side farther away from the inflow section can be avoided, and the attachment lugs in the first valve prosthesis 500 can therefore be prevented from interacting with the second valve prosthesis 600 or with the delivery device for delivering the second valve prosthesis 600.

Of course, in other embodiments, the angle at which the attachment portion 321 is bent and folded toward the interior of the stent body 310 may be alternatively greater than or equal to the angle at which the connection rod 322 is bent and folded toward the interior of the stent body 310.

In this embodiment, the connection rod includes a first connecting section 324 and a second connecting section 325. The first connecting section 324 is connected at one end to the stent body 310 and connected at the other end to one end of the second connecting section 325. Here, the end of the first connecting section 324, where it is connected to the stent body 310, forms the above-described first connecting end 320a. The other end of the second connecting section 325 is connected to the attachment portion.

The first connecting section 324 forms the above-described first bent section of the attachment lug 320, and the second connecting section 325 forms the above-described second bent section thereof. The first and second connecting sections 324, 325 are bent and folded toward the interior of the stent body 310 at angles that may be equal to not to each other. For example, the angle at which the second connecting section 325 is bent and folded toward the interior of the stent body 310 may be greater than the angle at which the first connecting section 324 is bent and folded toward the interior of the stent body 310. Alternatively, the angle at which the second connecting section 325 is bent and folded toward the interior of the stent body 310 may be less than the angle at which the first connecting section 324 is bent and folded toward the interior of the stent body 310.

As shown in Figs. 16 and 17, preferably, the second connecting section 325 and the attachment portion 321 together form the second bent section of the attachment lug. That is, the angle at which the first connecting section 324 is bent and folded toward the interior of the stent body 310 is greater than the angle at which the second connecting section 325 is bent and folded toward the interior of the stent body 310, and the angle at which the second connecting section 325 is bent and folded toward the interior of the stent body 310 is equal to the angle at which the attachment portion 321 is bent and folded toward the interior of the stent body 310. In other words, the attachment lug 320 is bent and folded at the junction between the first connecting section 324 and the second connecting section 325 so that the second connecting section 325 and attachment portion 321 are inclined away from the center axis of the stent body 310 at a predetermined angle with respect to the first connecting section 324.

Preferably, the angle at which the first bent section is bent and folded toward the interior of the stent body 310 ranges from 10° to 45°, and the angle at which the second bent section is bent and folded toward the interior of the stent body 310 ranges from 5° to 40°. More preferably, the angle at which the second bent section is bent and folded toward the interior of the stent body 310 ranges from 15° to 30°.

### Embodiment 3

In this embodiment, there is provided a valve prosthesis. Fig. 19 shows a front view of the valve prosthesis 100, which differs from the valve prosthesis 100 of Embodiment 1 essentially in that the attachment lugs 320 in the valve prosthesis 100 according to this embodiment are made through braiding.

Preferably, in this embodiment, the attachment lugs 320 are made of multiple braided layers.

The braided attachment lugs 320 according to this embodiment are softer than those fabricated otherwise, for example, by cutting. As a result, when the attachment lugs 320 are brought into contact with the wall of the aorta D, they can easily bend and folded toward the interior of the stent body 310 under the action of the compressive contact, without causing damage to the wall of the aorta D. Therefore, the risk of puncturing the wall of the aorta D by the attachment lugs 320 can be reduced.

In addition, the multi-layered braided attachment lugs 320 have a greater thickness than those fabricated by cutting, which can facilitate the attachment of the attachment lugs 320 to the delivery device 400, making it easier to load the valve prosthesis 100 into the delivery device 400. In addition, since the multi-layered braided attachment lugs 320 in the valve prosthesis 100 are softer, they can be more easily compressed and secured to the delivery device 400 during the loading of the valve prosthesis 100 into the sheath 410, additionally shortening the time required to load the valve stent 300 and resulting in improved loading efficiency.

With reference to Fig. 19, in this embodiment, both the stent body 310 and attachment lugs 320 in the valve prosthesis 100 may be fabricated by braiding. The attachment lugs 320 may be integral with the stent body 310 through braiding. Alternatively, the attachment lugs 320 and the stent body 310 may be separately braided, followed by welding the attachment lugs 320 to the stent body 310. In other embodiments, the stent body 310 of the valve prosthesis 100 may also be fabricated otherwise than by braiding.

In this embodiment, the attachment lugs 320 may be shaped using a mold so that they are bent and folded toward the interior of the stent body 310.

As shown in Fig. 19, each attachment lugs 320 may be L-shaped. That is, the attachment lug 320 may extend first toward the interior of the stent body 310 along a radial direction of the stent body 310 and then along the axial direction of the stent body 310 away from the outflow section 313.

Therefore, this embodiment differs from Embodiment 1 in that the attachment lugs 320 according to this embodiment may extend parallel to the axial direction of the stent body 310 away from the outflow section 313.

### Embodiment 4

In this embodiment, there is provided a valve prosthesis, which differs from the valve prosthesis of Embodiment 1 essentially in that the attachment lugs may be fabricated by cutting, with the stent body being fabricated by braiding.

### Embodiment 5

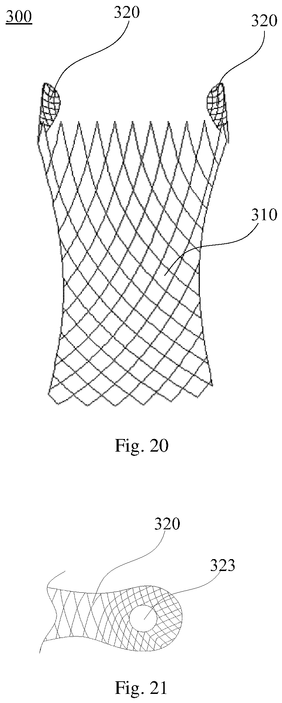

In this embodiment, there is provided a valve prosthesis, and reference is now made to Fig. 20, a front view of the valve prosthesis, and to Fig. 21, an enlarged view of part of an attachment lug in the valve prosthesis. The valve prosthesis according to this embodiment differs from that of Embodiment 1 essentially in that the attachment lugs 320 in the valve prosthesis 100 according to this embodiment may be fabricated by braiding.

Referring to Figs. 20 and 21, according to this embodiment, each attachment lug 320 defines a through hole 323 preferably located at a center of the attachment portion 321 of the attachment lug 320. The attachment lug 320 has a thickness preferably greater than a thickness of the stent body 310.

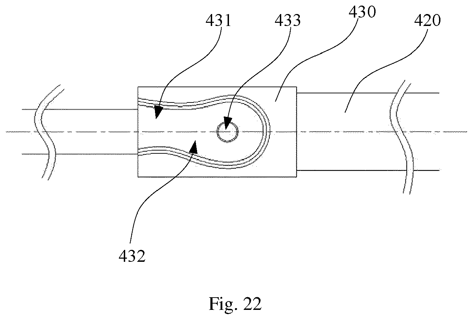

Reference is now made to Fig. 22, an enlarged view of part of a delivery device according to this embodiment. A protrusion 433 may be provided in the bottom portion 432 of each recess 431 in the attachment member 430 of the delivery device 400. Preferably, the through hole 323 of each attachment lug 320 is complementary in shape to a respective one of the protrusions 433. In order to load the valve prosthesis 100 into the delivery device 400, the valve prosthesis 100 may be slightly compressed so that the attachment lugs 320 are inwardly deflected, leading to insertion of the protrusions 433 in the bottom portions 432 of the respective recesses 431 in the attachment member 430 into the through holes in the respective attachment lugs 320. This means that even a slight compression of the attachment lugs 320 in the valve prosthesis 100 can bring them into contact with the bottom portions 432 of the respective recesses 431 in the attachment member 430, which can facilitate the loading of the attachment lugs 320 into the sheath 410.

In the valve stent, valve prosthesis and delivery device provided herein, the bending and folding of the attachment lugs toward the interior of the stent body can offer the following advantages:
First, bending and folding the attachment lugs toward the interior of the stent body can reduce the difficulty in inwardly deflecting the attachment lugs and securing them to the delivery device during the valve prosthesis's loading into the sheath of the delivery device and can thus shorten the time required for the loading of the valve stent, resulting in increased valve prosthesis loading efficiency.

Second, the angle at which the attachment lugs are bent and folded toward the interior of the stent body is greater than the angle at which an end of the outflow section farther away from the intermediate section is bent and folded toward the interior thereof, thus additionally lowering the difficulty in inwardly deflecting the attachment lugs and securing them to the delivery device during the valve prosthesis's loading into the sheath of the delivery device, additionally shortening the time required for the loading of the valve stent and resulting in additional increase in valve prosthesis loading efficiency.

Third, bending and folding the attachment lugs toward the interior of the stent body can lower the risk of puncturing the ascending aortic wall by the attachment lugs.

Fourth, bending and folding the attachment lugs in valve prosthesis toward the interior of the stent body can limit the axial movement of the second valve prosthesis in a ViV procedure, reducing the risk of valve prosthesis displacement.

The description presented above is merely that of a few preferred embodiments of the present application and does not limit the scope thereof in any sense. Any of changes and modifications made by those of ordinary skill in the art based on the above teachings falls within the scope as defined in the appended claims.

## Claims

1. A valve stent (300), comprising:
a stent body (310) shaped as a tubular mesh; and
at least two attachment lugs (320), each connected to the stent body (310) and bent and folded toward an interior of the stent body (310);
**characterized in that** each of the attachment lugs (320) comprises a first bent section and a second bent section; the first bent section is bent and folded toward the interior of the stent body (310) at an angle greater than an angle at which the second bent section is bent and folded toward the interior of the stent body (310); the first bent section is located closer than the second bent section to the stent body (310), with respect to an axial direction of the stent body (310).

2. The valve stent (300) of claim 1, wherein the angle at which the first bent section is bent and folded toward the interior of the stent body (310) ranges from 10° to 45° and the angle at which the second bent section is bent and folded toward the interior of the stent body (310) ranges from 5° to 40°;
the angle at which the second bent section is bent and folded toward the interior of the stent body (310) ranges from 15° to 30°.

3. The valve stent (300) of claim 1, wherein the first bent section comprises a connection rod (322) and the second bent section comprises an attachment portion (321); the connection rod (322) has one end connected to the stent body (310) and a further end connected to the attachment portion (321); one end of the attachment portion (321) which is farther away from the connection rod (322) is an free end.

4. The valve stent (300) of claim 1, wherein the first bent section comprises a first connecting section (324) and the second bent section comprises an attachment portion (321) and a second connecting section (325) connected to the attachment portion (321); the second connecting section (325) is connected to one end of the first connecting section (324), a further end of the first connecting section (324) is connected to the stent body (310), one of the attachment portion (321) which is farther away from the second connecting section (325) is a free end.

5. The valve stent (300) of any one of claims 1 to 4, wherein the at least two attachment lugs (320) have a same shape and are uniformly distributed circumferentially on the stent body (310).

6. The valve stent (300) of any one of claims 1 to 4, wherein an attachment portion (321) of each of the attachment lugs (320) defines a through hole (323).

7. The valve stent (300) of any one of claims 1 to 4, wherein each of the attachment lugs (320) has a length ranging from 1 mm to 6 mm, in particular, from 3 mm to 5 mm.

8. The valve stent (300) of any one of claims 1 to 4, wherein the attachment lugs (320) are fabricated through cutting or braiding.

9. A valve prosthesis (100), comprising an artificial valve (200) and a valve stent (300) as claimed in any one of claims 1 to 8, wherein the artificial valve (200) is attached to the valve stent (300).

10. A delivery device (400), configured to deliver the valve prosthesis (100) of claim 9, the delivery device (400) comprising:
a sheath (410) configured to receive the valve prosthesis (100);
an inner tube (420) disposed inside the sheath (410); and
an attachment member (430) sleeved over and fixed to the inner tube (420), the attachment member (430) configured to restrict positions of the attachment lugs (320) in the valve prosthesis (100) relative to the inner tube (420).

11. The delivery device (400) of claim 10, further comprising a guide tube (440) disposed outside the sheath (410), wherein the guide tube (440) has a tapered inner surface (411) and is able to be sleeved over the inner tube (420), the guide tube (440) is configured to clamp the attachment lugs (320) of the valve prosthesis (100) against the attachment member (430).

12. The delivery device (400) of claim 10, wherein a plurality of recesses (431) are formed in an outer circumferential surface of the attachment member (430) in a uniform manner, and each of the recesses (431) is configured to receive the attachment portion (321) of a respective one of the attachment lugs (320).

13. The delivery device (400) of claim 12, wherein the recess (431) comprises a bottom portion (432) complementary in shape to the attachment portion (321).

14. The delivery device (400) of claim 13, wherein a protrusion (433) is provided in the bottom portion (432) of the recess (431), and the protrusion (433) matches with a through hole (323) defined in the attachment portion (321) of the respective attachment lug (320) in the valve prosthesis (100).

## Patentansprüche

1. Klappenstent (300), umfassend:
einen Stentkörper (310) in Form eines röhrenförmigen Netzes; und
mindestens zwei Befestigungslaschen (320), die jeweils mit dem Stentkörper (310) verbunden und zu einem Inneren des Stentkörpers (310) hin gebogen und gefaltet sind;
**dadurch gekennzeichnet, dass** jede der Befestigungslaschen (320) einen ersten gebogenen Abschnitt und einen zweiten gebogenen Abschnitt umfasst; der erste gebogene Abschnitt ist in Richtung des Inneren des Stentkörpers (310) in einem Winkel gebogen und gefaltet, der größer ist als ein Winkel, in dem der zweite gebogene Abschnitt in Richtung des Inneren des Stentkörpers (310) gebogen und gefaltet ist; der erste gebogene Abschnitt ist in Bezug auf eine axiale Richtung des Stentkörpers (310) näher als der zweite gebogene Abschnitt an dem Stentkörper (310) angeordnet.

2. Klappenstent (300) nach Anspruch 1, wobei der Winkel, in dem der erste gebogene Abschnitt in Richtung des Inneren des Stentkörpers (310) gebogen und gefaltet ist, im Bereich von 10° bis 45° liegt und der Winkel, in dem der zweite gebogene Abschnitt in Richtung des Inneren des Stentkörpers (310) gebogen und gefaltet ist, im Bereich von 5° bis 40° liegt;
der Winkel, in dem der zweite gebogene Abschnitt in Richtung des Inneren des Stentkörpers (310) gebogen und gefaltet ist, liegt zwischen 15° und 30°.

3. Klappenstent (300) nach Anspruch 1, wobei der erste gebogene Abschnitt einen Verbindungsstab (322) und der zweite gebogene Abschnitt einen Befestigungsabschnitt (321) umfasst; der Verbindungsstab (322) hat ein Ende, das mit dem Stentkörper (310) verbunden ist, und ein weiteres Ende, das mit dem Befestigungsabschnitt (321) verbunden ist; ein Ende des Befestigungsabschnitts (321), das weiter von dem Verbindungsstab (322) entfernt ist, ist ein freies Ende.

4. Klappenstent (300) nach Anspruch 1, wobei der erste gebogene Abschnitt einen ersten Verbindungsabschnitt (324) umfasst und der zweite gebogene Abschnitt einen Befestigungsabschnitt (321) und einen zweiten Verbindungsabschnitt (325) umfasst, der mit dem Befestigungsabschnitt (321) verbunden ist; der zweite Verbindungsabschnitt (325) mit einem Ende des ersten Verbindungsabschnitts (324) verbunden ist, ein weiteres Ende des ersten Verbindungsabschnitts (324) mit dem Stentkörper (310) verbunden ist, einer der Befestigungsabschnitte (321), der weiter von dem zweiten Verbindungsabschnitt (325) entfernt ist, ein freies Ende ist.

5. Klappenstent (300) nach einem der Ansprüche 1 bis 4, wobei die mindestens zwei Befestigungslaschen (320) eine gleiche Form aufweisen und gleichmäßig in Umfangsrichtung auf dem Stentkörper (310) verteilt sind.

6. Klappenstent (300) nach einem der Ansprüche 1 bis 4, wobei ein Befestigungsabschnitt (321) jeder der Befestigungslaschen (320) ein Durchgangsloch (323) definiert.

7. Klappenstent (300) nach einem der Ansprüche 1 bis 4, wobei jede der Befestigungslaschen (320) eine Länge von 1 mm bis 6 mm, insbesondere von 3 mm bis 5 mm, aufweist.

8. Klappenstent (300) nach einem der Ansprüche 1 bis 4, wobei die Befestigungslaschen (320) durch Schneiden oder Flechten hergestellt werden.

9. Klappenprothese (100), umfassend eine künstliche Klappe (200) und einen Klappenstent (300) nach einem der Ansprüche 1 bis 8, wobei die künstliche Klappe (200) mit dem Klappenstent (300) verbunden ist.

10. Zuführungsvorrichtung (400), die zum Zuführen zur Klappenprothese (100) nach Anspruch 9 konfiguriert ist, wobei die Zuführungsvorrichtung (400) umfasst:
eine Hülle (410), die zur Aufnahme der Klappenprothese (100) konfiguriert ist;
ein Innenrohr (420), das im Inneren der Hülle (410) angeordnet ist; und
ein Befestigungselement (430), das über das Innenrohr (420) gestülpt und daran befestigt ist, wobei das Befestigungselement (430) so konfiguriert ist, dass es die Positionen der Befestigungslaschen (320) in der Ventilprothese (100) im Verhältnis zum Innenrohr (420) begrenzt.

11. Zuführungsvorrichtung (400) nach Anspruch 10, die ferner ein Führungsrohr (440) umfasst, das außerhalb der Hülle (410) angeordnet ist, wobei das Führungsrohr (440) eine sich verjüngende Innenfläche (411) aufweist und über das Innenrohr (420) gestülpt werden kann, wobei das Führungsrohr (440) so konfiguriert ist, dass es die Befestigungslaschen (320) der Ventilprothese (100) gegen das Befestigungselement (430) klemmt.

12. Zuführungsvorrichtung (400) nach Anspruch 10, wobei eine Vielzahl von Aussparungen (431) in einer äußeren Umfangsfläche des Befestigungselements (430) in gleichmäßiger Weise ausgebildet sind und jede Aussparung (431) so konfiguriert ist, dass sie den Befestigungsabschnitt (321) jeweils einer der Befestigungslaschen (320) aufnimmt.

13. Zuführungsvorrichtung (400) nach Anspruch 12, wobei die Aussparung (431) einen unteren Abschnitt (432) umfasst, der ergänzend zum Befestigungsabschnitt (321) geformt ist.

14. Zuführungsvorrichtung (400) nach Anspruch 13, wobei ein Vorsprung (433) im unteren Abschnitt (432) der Aussparung (431) vorgesehen ist und der Vorsprung (433) mit einem Durchgangsloch (323) zusammenpasst, das im Befestigungsabschnitt (321) der jeweiligen Befestigungslasche (320) in der Klappenprothese (100) definiert ist.

## Revendications

1. Endoprothèse valvulaire (300), comprenant :
un corps d'endoprothèse (310) en forme de filet tubulaire ; et
au moins deux pattes de fixation (320), chacune reliée au corps d'endoprothèse (310) et courbée et pliée vers l'intérieur du corps d'endoprothèse (310) ;
**caractérisée en ce que** chacune des pattes de fixation (320) comprend une première section courbée et une seconde section courbée ; la première section courbée est courbée et pliée vers l'intérieur du corps d'endoprothèse (310) à un angle supérieur à un angle auquel la seconde section courbée est courbée et pliée vers l'intérieur du corps d'endoprothèse (310) ; la première section courbée est située plus près que la seconde section courbée du corps d'endoprothèse (310), par rapport à une direction axiale du corps d'endoprothèse (310).

2. Endoprothèse valvulaire (300) selon la revendication 1, dans laquelle l'angle auquel la première section courbée est courbée et pliée vers l'intérieur du corps d'endoprothèse (310) est dans la plage de 10° à 45° et l'angle auquel la seconde section courbée est courbée et pliée vers l'intérieur du corps d'endoprothèse (310) est dans la plage de 5° à 40° ;
l'angle auquel la seconde section courbée est courbée et pliée vers l'intérieur du corps d'endoprothèse (310) est dans la plage de 15° à 30°.

3. Endoprothèse valvulaire (300) selon la revendication 1, dans laquelle la première section courbée comprend une tige de liaison (322) et la seconde section courbée comprend une partie de fixation (321) ; la tige de liaison (322) a une extrémité reliée au corps d'endoprothèse (310) et une autre extrémité reliée à la partie de fixation (321) ; une extrémité de la partie de fixation (321) qui est la plus éloignée de la tige de liaison (322) est une extrémité libre.

4. Endoprothèse valvulaire (300) selon la revendication 1, dans laquelle la première section courbée comprend une première section de liaison (324) et la seconde section courbée comprend une partie de fixation (321) et une seconde section de liaison (325) reliée à la partie de fixation (321) ; la seconde section de liaison (325) est reliée à une extrémité de la première section de liaison (324), une autre extrémité de la première section de liaison (324) est reliée au corps d'endoprothèse (310), l'une de la partie de fixation (321) qui est la plus éloignée de la seconde section de liaison (325) est une extrémité libre.

5. Endoprothèse valvulaire (300) selon l'une quelconque des revendications 1 à 4, dans laquelle les au moins deux pattes de fixation (320) ont la même forme et sont uniformément réparties de manière circonférentielle sur le corps d'endoprothèse (310).

6. Endoprothèse valvulaire (300) selon l'une quelconque des revendications 1 à 4, dans laquelle une partie de fixation (321) de chacune des pattes de fixation (320) définit un trou traversant (323).

7. Endoprothèse valvulaire (300) selon l'une quelconque des revendications 1 à 4, dans laquelle chacune des pattes de fixation (320) a une longueur dans la plage de 1 mm à 6 mm, en particulier de 3 mm à 5 mm.

8. Endoprothèse valvulaire (300) selon l'une quelconque des revendications 1 à 4, dans laquelle les pattes de fixation (320) sont fabriquées par découpage ou tressage.

9. Prothèse valvulaire (100), comprenant une valve artificielle (200) et une endoprothèse valvulaire (300) selon l'une quelconque des revendications 1 à 8, dans laquelle la valve artificielle (200) est fixée à l'endoprothèse valvulaire (300).

10. Dispositif d'administration (400), configuré pour administrer la prothèse valvulaire (100) selon la revendication 9, le dispositif d'administration (400) comprenant :
une gaine (410) configurée pour recevoir la prothèse valvulaire (100) ;
un tube intérieur (420) disposé à l'intérieur de la gaine (410) ; et
un élément de fixation (430) manchonné sur le tube intérieur (420) et fixé à celui-ci, l'élément de fixation (430) étant configuré pour restreindre les positions des pattes de fixation (320) dans la prothèse valvulaire (100) par rapport au tube intérieur (420).

11. Dispositif d'administration (400) selon la revendication 10, comprenant en outre un tube de guidage (440) disposé à l'extérieur de la gaine (410), dans lequel le tube de guidage (440) a une surface intérieure conique (411) et peut être manchonné sur le tube intérieur (420), le tube de guidage (440) est configuré pour serrer les pattes de fixation (320) de la prothèse valvulaire (100) contre l'élément de fixation (430).

12. Dispositif d'administration (400) selon la revendication 10, dans lequel une pluralité d'évidements (431) sont formés de manière uniforme dans une surface circonférentielle extérieure de l'élément de fixation (430), et chacun des évidements (431) est configuré pour recevoir la partie de fixation (321) d'une des pattes de fixation (320) respective.

13. Dispositif d'administration (400) selon la revendication 12, dans lequel l'évidement (431) comprend une partie inférieure (432) de forme complémentaire à la partie de fixation (321).

14. Dispositif d'administration (400) selon la revendication 13, dans lequel une protubérance (433) est prévue dans la partie inférieure (432) de l'évidement (431), et la protubérance (433) correspond à un trou traversant (323) défini dans la partie de fixation (321) de la patte de fixation (320) respective dans la prothèse valvulaire (100).
